# EUROPEAN PATENT SPECIFICATION

(11) **EP 2 175 869 B1**
(45) Date of publication and mention of the grant of the patent: **19.12.2012**
(21) Application number: 08785481.6
(22) Date of filing: 11.08.2008
(51) Int. Cl.: A61K 35/32, A61K 38/18, A61P 19/00

(54) **Compositions for repair of lesions in cartilage and bone**
Zusammensetzungen zur Reparatur von Läsionen in Knorpel und Knochen
Compositions pour la réparation de lésions cartilagineuses et osseuses

(30) Priority: 14.08.2007 EP 07405236
(43) Date of publication of application: 21.04.2010
(73) Proprietor: University of Bern, 3010 Bern (CH)
(72) Inventor: SHINTANI, Nahoko, 3008 Bern (CH)
(74) Representative: Schmauder & Partner AG Patent- & Markenanwälte VSP
(86) International application number: PCT/EP2008/006601
(87) International publication number: WO 2009/021704

(56) References cited:
- SHIRASAWA ET AL: "In vitro chondrogenesis of human synovium-derived mesenchymal stem cells: Optimal condition and comparison with bone marrow-derived cells" JOURNAL OF CELLULAR BIOCHEMISTRY, vol. 97, 2006, pages 84-97, XP003021090
- SHINTANI ET AL: "Chondrogenic differentiation of bovine synovium" ARTHRITIS & RHEUMATISM, vol. 56, June 2007 (2007-06), pages 1869-1879, XP002503857
- NIIKURA ET AL: "Differential regulation of lubricin/superficial zone protein by transforming growth factor beta/bone morphogenetic protein superfamily members in articular chondrocytes and synoviocytes" ARTHRITIS & RHEUMATISM, vol. 56, July 2007 (2007-07), pages 2312-2321, XP002503858
- PEI ET AL: "Engineering of functional cartilage tissue using stem cells from synovial lining" CLINICAL ORTHOPAEDICS AND RELATED RESEARCH, vol. 466, 30 May 2007 (2007-05-30), pages 1880-1889, XP002503893
- KURTH ET AL: "Chondrogenic potential of human synovial mesenchymal stem cells in alginate" OSTEOARTHRITIS AND CARTILAGE, vol. 15, 14 May 2007 (2007-05-14), pages 1178-1189, XP022237903
- YAMAZAKI ET AL: "Vascularized synovial flap promoting regeneration of the cryopreserved meniscal allograft: experimental study in rabbits" JOURNAL OF ORTHOPAEDIC SCIENCE, vol. 8, 2003, pages 62-68, XP002503859
- GOLDING: "Update on the biology of the chondrocyte and new approaches to treating cartilage diseases" BEST PRACTICE & RESEARCH CLINICAL RHEUMATOLOGY, vol. 20, 2006, pages 1003-1025, XP005652470
- SHINTANI ET AL: "Transforming growth factor beta 1 enhances and modifies the bone-morphogenetic -protein-2-induced chondrogenic differentiation of bovine synovial-tissue explants" OSTEOARTHRITIS AND CARTILAGE, vol. 15, 5 September 2007 (2007-09-05), page B72, XP022221460
- "Chapter 10: Regenerative medicine of musculoskeletal tissues" In: Stocum: "Regenerative biology and medicine", 2006, Elsevier / Academic Press, XP008137926, pages 251-281,

## Description

### Field of the invention

This invention relates to compositions for the treatment and repair of defects or lesions in cartilage and bone.

### Background of the invention

Joints are one of the common ways bones in the skeleton are connected. The ends of normal articulated bone are covered by articular cartilage tissue, which permits practically frictionless movement of the bones with respect to one another.

Articular cartilage is characterized by a particular structural organization. It consists of specialized cells, so-called chondrocytes, embedded in an intercellular material often referred to in the literature as the "cartilage matrix", which is rich in proteoglycans, collagen fibrils of predominantly type II, other proteins, and water [Buckwalter et al., "Articular Cartilage: Injury and Repair", in Injury and Repair of the Musculoskeletal Soft Tissues (Park Ridge, III.: American Academy of Orthopaedic Surgeons Symposium, 1987) p. 465]. Cartilage tissue is neither innervated nor penetrated by the vascular or lymphatic systems. However, in the mature joint of adults, the underlying subchondral bone tissue, which forms a narrow, continuous plate between the bone tissue and the cartilage, is innervated and vascularized. Beneath this bone plate, the bone tissue forms trabeculae, containing the marrow. In immature joints, articular cartilage is underlined by only primary bone trabeculae. A portion of the meniscal tissue in joints also consists of cartilage whose make-up is similar to articular cartilage.

Two types of defects are recognized in articular surfaces, i.e., full-thickness defects and superficial defects. These defects differ not only in the extent of physical damage to the cartilage, but also in the nature of the repair response each type of lesion can elicit.

Full-thickness defects of an articular surface include damage to the hyaline cartilage, the calcified cartilage layer and the subchondral bone tissue with its blood vessels and bone marrow. The damage to the bone tissue may range from a fissure or crack to an enlarged gap in the bone tissue. Full-thickness defects can cause severe pain since the bone plate contains sensory nerve endings. Such defects generally arise from severe trauma or during the late stages of degenerative joint disease, such as osteoarthritis. Full-thickness defects may, on occasion, lead to bleeding and the induction of a repair reaction from the subchondral bone [Buckwalter et al., "Articular Cartilage and Knee Joint Function: Basic Science and Arthroscopy" (New York: Raven Press, 1990) p. 19-56]. The repair tissue formed is a vascularized fibrous type of cartilage with insufficient biomechanical properties, and does not persist on a long-term basis.

Superficial defects in the articular cartilage tissue are restricted to the cartilage tissue itself. Such defects are notorious because they do not heal and show no propensity for repair reactions. Superficial defects may appear as fissures, divots, or clefts in the surface of the cartilage, or they may have a "crabmeat" appearance in the affected tissue. They contain no bleeding vessels (blood spots) such as are seen in full-thickness defects. Superficial defects may have no known cause, but often they are the result of mechanical derangements which lead to a wearing down of the cartilaginous tissue. Mechanical derangements may be caused by trauma to the joint, e.g., a displacement of torn meniscus tissue into the joint, meniscectomy, a laxation of the joint by a torn ligament, malalignment of joints, or bone fracture, or by hereditary diseases. Superficial defects are also characteristic of early stages of degenerative joint diseases, such as osteoarthritis. Since the cartilage tissue is not innervated or vascularized, superficial defects are not painful. However, although painless, superficial defects do not heal and often degenerate into full-thickness defects.

Millions of patients have been diagnosed as having osteoarthritis, i.e., as having degenerating defects or lesions in their articular cartilage. Nevertheless, despite claims of various surgical treatments to elicit a repair response in damaged cartilage, none of these treatments has received substantial application, and such treatments have generally provided only temporary relief [Siparsky P. et al., Clin. Orthop. 455, 107 (2007)]. Systemic use of "chondro-protective agents" has also been purported to arrest the progression of osteoarthritis and to induce relief of pain. However, such agents have not been shown to promote repair of lesions or defects in cartilage tissue [Reginster et al., Lancet 357, 251 (2001), Shikhman et al., Ann. Rheum. Dis. 64, 89 (2005)].

To date, treatment of patients suffering from osteoarthritis is directed largely to symptomatic relief through the use of analgesics and anti-inflammatory agents. Without a treatment that will elicit repair of superficial defects in articular cartilage, the cartilage frequently wears down to the subchondral bone plate. At this phase of the disease, i.e., severe osteoarthritis, the unremitting nature on the pain and the significant compromise of function often dictates that the entire joint be excised and replaced with an artificial joint of metal and/or plastic. Some one-half million procedures comprising joint resection and replacement with an artificial joint are currently performed on knees and hips each year. There is, therefore, a need for a reliable treatment for cartilage tissue in superficial cartilage defects and for cartilage and bone tissue in full-thickness defects, e.g., as found in cases of severe osteoarthritis.

It is generally believed that because articular cartilage lacks a vasculature, damaged cartilage tissue does not receive sufficient or proper stimuli to elicit a repair response. It is theorized that the chondrocytes in the cartilaginous tissue are normally not exposed to sufficient amounts of repair-stimulating agents such as growth factors and fibrin clots typically present in damaged vascularized tissue.

One approach that has been used to expose damaged cartilage tissue to repair stimuli involves drilling or scraping through the cartilage into the subchondral bone to cause bleeding. Unfortunately, the repair response of the tissue to such surgical trauma is usually comparable to that observed to take place naturally in full-thickness defects that cause bleeding, viz., formation of a fibrous type of cartilage which exhibits insufficient biomechanical properties and which does not persist on a long-term basis [Buckwalter et al. (1990), supra].

An increasing number of research teams around the world are now adopting a more biologically rational approach to the repair of articular cartilage lesions. These activities are directed mainly towards elaborating novel tissue-engineering-based strategies to generate substitute cartilage at the defect site. A commonly tested model is represented by chondroprogenitor cells embedded within a matrix. The chondroprogenitor cells used for such purposes can be of diverse origin. They can be derived from articular cartilage itself or from various tissues containing mesenchymal stem cells, such as bone marrow, periosteum, perichondrium, muscle, fat and synovium [Caplan A.I., Tissue Eng. 1198-1211 (2005)]. A variety of growth factors have been isolated and are now available for research and biomedical applications [see e.g., Wozney J.M. et al., Curr. Opin. Biotechnol. 392-398 (2004)]. Some of these growth factors, such as bone morphogenic protein (BMP) 2, 4, 6 and 7 or a combination of transforming growth factor beta (TGF-ß) and dexamethasone, have been reported to promote formation of cartilage-specific molecules, such as type II collagen and cartilage-specific proteoglycans, in mesenchymal stem cells derived from various tissues *in vitro.* However, these growth factors also induce the expression of type X collagen, which is a marker of terminal (hypertrophic) differentiation of chondrocytes. Hypertrophic chondrocytes can proceed into ossification. In fact, most of these growth factors have both a chondrogenic and osteogenic potential. Recently, it has been reported that terminal differentiation of chondrocytes can be regulated with various factors [TGF-ß/Smad3: Yang X., J. Cell Biol. 35-46 (2001); PTHrP: Minina E., Development 4523-4534 (2001); Wnt5a and Wnt5b: Church V. J., Cell Sci. 4809-4818 (2002); Smad6 and Smad7: Valcourt U., J. Biol. Chem. 33545-33558 (2002); Smad6/Smurf1: Horiki M., J. Cell Biol. 433-445 (2004); Wnt: Dong Y., J. Cell Biochem. 1057-1068 (2005); E2F1, TGF-ß, PTHrP, Indian hedghog, Wnt5b, ILK, ß1 integrin: Beier F., J. Cell. Physiol. 1-8 (2005); Mrf1: Amano K., Abstract of 28th annual meeting of American society for bone and mineral research (2006)].

One of the most important issues of cartilage repair using growth factors and chondroprogenitor cells is how to prevent the terminal differentiation of the chondrocytes which were induced by the growth factors. However, so far, there are no established treatments for defects and lesions in cartilage on the basis of this perspective. Actually, TGF-ß1 is shown to modulate the terminal differentiation of the periosteal cells stimulated with BMP-2 [Hanada et al., J. Cell. Biochem. 81, 284 (2001)].

Synovium is known to contain mesenchymal stem cells and these cells differentiate into chondrocytes under appropriate stimulation conditions [De Bari et al., Arthritis Rheum. 44, 85 (2001)]. Recently, it has been shown that mesenchymal stem cells derived from synovium have higher chondrogenic potential than those derived from bone marrow, adipose, periosteum and muscle [Sakaguchi et al., Arthritis Rheum. 52, 2521 (2005)]. In these reports the authors used BMP-2 alone, TGF-ß1 alone, combination with TGF-ß1 and dexamethasone, or combination with TGF-ß3, BMP-2 and dexamethasone to induce chondrogenesis of synovial cells. However, no one considered how to control the terminal differentiation of the chondrocytes transformed from synovial cells.

Shirasawa et al., Journal of Cellular Biochemistry 97, 84-97 (2006), XP003021090, describes experiments carried out with synovial cell pellets. Such pellets were obtained from human synovium, which was minced and digested with Collagenase D; the synovial cells thus obtained were plated in a dish with culture medium, expanded, replated, incubated and then harvested. They were then cryopreserved in liquid nitrogen. Later they were thawed, plated and incubated again in a recovery plate. Finally, cell pellets were formed by centrifugation. The authors found that in order to achieve faster growth of synovial cells aggregated in a pellet, it is better to use a combination of TGFß, dexamethasone and BMP2. Shintani et al., Arthritis and Rheumatism 56, 1869-1879 (2007), XP002503857, studied chondrogenic differentiation of bovine synovium by studying the growth behavior of micromass cell cultures exposed to various growth factors. Shortly thereafter, Niikura et al., Arthritis and Rheumatism 56, 2312-2321 (2007), XP002503858, published a study concerning the influence of various growth factors in cultures of chondrocytes and synoviocytes. However, all these investigations did not address the problem of terminal differentiation that arises when exposing tissue flaps to various growth factors.

Yamazaki et al., Journal of Orthopaedic Science 8, 62-68 (2003), XP002503859, studied the regeneration of cryopreserved meniscal allograft by application of vascularized synovial flaps.

Goldring M.B., Best Practice & Research Clinical Rheumatology 20, 1003-1025 (2006), XP005652470, generally describes various approaches to treating cartilage diseases.

The use of synovial flaps for the treatment of meniscus (cartilage) problems in general has been described in a review by Stocum D.L., Regenerative Biology and Medicine, 251-281 (2006), XP008137926.

There is, therefore, a need for a reliable and improved treatment for cartilage tissue in superficial cartilage defects and for cartilage and bone tissue in full-thickness defects, e.g., as found in cases of severe osteoarthritis.

### Summary of the invention

The present invention provides compositions to induce the repair of lesions in cartilage and bone of animals, in particular humans. The compositions of this invention also promote the healing of traumatic lesions and forms of osteoarthritis, which would otherwise lead to loss of effective joint function leading to probable resection and replacement of the joint.

The method for treating superficial cartilage defects or the cartilage portion of full-thickness defects comprises filling the cartilage portion of the defect with layers of flaps of synovium or peritendineum with interposed layers of the cartilage repair matrix containing a chondrogenic factor for inducing chondrogenesis and an anti-hypertrophic agent for preventing hypertrophic differentiation. In full-thickness defects, the layer between bone and cartilage may serve as a membranous barrier to the upgrowth of bone and blood vessels into the cartilaginous defect compartment. The cartilage repair matrix will be incorporated into the animal or human tissue and is generally biodegradable. The invention provides compositions useful in this method comprising peritendineal tissue or synovial tissue, one or more chondrogenic factors, and one or more anti-hypertrophic agents. In particular, such compositions comprise layers of flaps of synovium or peritendineum with interposed layers of the cartilage repair matrix containing a chondrogenic factor and an anti-hypertrophic agent.

It was found that peritendineum contains chondroprogenitor cells, as it is known for synovium. Peritendineum transdifferentiates into cartilaginous tissue under stimulation conditions similar to those used to stimulate synovium. As a consequence, peritendinuem and synovium are equally useful for the treatment and repair of defects or lesions in cartilage or bone.

The method may comprise heat-treating the areas in a full-thickness defect where bleeding has occurred to create a transient tissue barrier and then fill the defect with a cartilage repair matrix and layers of flaps of synovium or peritendineum. In the bone area, anti-hypertrophic agent may be omitted from the matrix.

The method for repairing full-thickness defects in joints also comprises, where necessary or desirable due to extensive bone injury, filling the defect in the bone portion of a full-thickness defect up to the level of the bone-cartilage interface with a matrix that will be incorporated into animal or human tissue and is generally biodegradable. If the bone defect is large, layers of flaps of synovium or peritendineum may also be introduced into the bone defect. The bone repair matrix may contain osteogenic factors but no anti-hypertrophic agent. The corresponding compositions in the kit of the invention for bone repair comprise layers of flaps of synovium or peritendineum with interposed layers of the bone repair matrix containing a chondrogenic factor and an osteogenic factor. The remaining cartilage portion of the defect is filled to the top of the cartilage surface with cartilage repair matrix containing chondrogenic factor and anti-hypertrophic agent and layers of flaps of synovium or peritendineum.

The method may comprise enzyme-treating or needle-punching the flaps of synovium or peritendineum to enhance the chondrogenic and osteogenic potential of the flaps.

Treatment of superficial and full-thickness defects can be effected during arthroscopic, open surgical or percutaneous procedures using the methods of this invention. According to certain methods, after identification of a full-thickness defect, the defect is treated by the steps of (1) taking synovium or peritendineum from the joint, (2) preparing flaps of these tissues and treating them with enzymes or punching them by a needle, (3) filling the bone portion of the defect with a matrix containing an osteogenic factor packaged into an appropriate delivery system, e.g., liposomes; and (4) filling the cartilage portion of the defect with layers of flaps of synovium or peritendineum interposed with a matrix, preferably biodegradable, containing a chondrogenic factor and an anti-hypertropic agent which are packaged into an appropriate delivery system. In this fourth step, the flaps and matrix may be bonded to the surface of the cartilage, for example, by using an adhesion-promoting factor, such as transglutaminase.

### Detailed description of the invention

Synovium (synovial tissue) - as used herein, refers to a connective tissue that has two layers (intima and subintima). Synovium is delimited from the joint cavity by intima layer. The intima consists of sheet cells (types A and B). The underlying subintima contains fibroblast-like (mesenchymal stem) cells, macrophages, adipocytes and blood vessels.

Peritendineum (peritendineal tissue) - as used herein, refers to a type of connective tissue investing larger tendons extending as septa between the fibers composing them. Mesenchymal stem cells - as used herein, refers to the formative pluripotent blast or embryonic-like cells found in various tissues, such as bone marrow, blood, dermis, periosteum, fat muscle, perichondrium and synovium that are capable of differentiating into specific types of mesenchymal or connective tissue including adipose, osseous, cartilaginous, elastic, muscular, and fibrous connective tissues. The specific differentiation pathway into which these cells enter depends upon various influences, e.g., mechanical influences and/or endogenous bioactive factors, such as growth factors, cytokines, and/or local microenvironmental conditions established by host tissues.

Chondroprogenitor cell - as used herein, refers to a cell which, when exposed to appropriate stimuli, will differentiate and be transformed into a chondrocyte. Chondroprogenitor cells include mesenchymal cells, fibroblasts, fibroblast-like cells, macrophages and dedifferentiated chondrocytes.

Osteoprogenitor cell - as used herein, refers to a cell which, when exposed to appropriate stimuli, will differentiate and be transformed into a bone cell, such as an osteoblast or an osteocyte, which forms bone. Osteoprogenitor cells include perivascular cells, mesenchymal cells, fibroblasts, fibroblast-like cells, macrophages and dedifferentiated chondrocytes.

Bone - as used herein, refers to a calcified connective tissue primarily comprising a network of deposited calcium phosphate in the form of hydroxyapatite, collagen (predominantly type I collagen) and bone cells, such as osteoblasts and osteoclasts.

Cartilage - as used herein, refers to a type of connective tissue that contains chondrocytes embedded in an intercellular material (often referred to as the "cartilage matrix") comprising fibrils of collagen (predominantly type II collagen along with other minor types, e.g., types IX and XI), various proteoglycans (e.g., chondroitinsulfate-, keratansulfate-, and dermatansulfate proteoglycans), other proteins, and water. Cartilage as used herein includes articular and meniscal cartilage. Articular cartilage covers the surfaces of the portions of bones in joints and allows movement in joints without direct bone-to-bone contact, and thereby prevents wearing down and damage to apposing bone surfaces. Most normal healthy articular cartilage is also described as "hyaline", i.e., having a characteristic frosted glass appearance. Meniscal cartilage is usually found in joints which are exposed to concussion as well as movement. Such locations of meniscal cartilage include the temporo-mandibular, sterno-clavicular, acromio-clavicular, wrist and knee joints.

Chondrocytes - as used herein, refers to cells which are capable of producing components of cartilage tissue, e.g., type II cartilaginous fibrils and fibers and proteoglycans.

Arthroscopy - as used herein, refers to the use of an arthroscope to examine or perform surgery on a joint.

Anti-hypertrophic agent - as used herein, refers to any peptide, polypeptide, protein, or any other compound or composition with biological activity that suppresses or inhibits that chondrocyes differentiate into hypertrophic chondrocytes. The ability of the compound or composition to suppress production of hypertrophic chondrocyte-characteristic type X collagen can be determined by an *in vitro* assay (e.g. RT-PCR). Examples of anti-hypertrophic agents are TGF-ßs, PTHrP, Wnt family proteins (proteins of Wnt5a or Wnt5b gene), Smad proteins (Smad3, Smad6 and Smad7) and MRF1 (protein of Mrf1 gene), prostaglandin E-2 (PGE-2), and transcription factors, such as AP-2, delta EF-1, P300, E2F1 and the like. Compositions of the invention contain one or more of these anti-hypertrophic agents, i.e. one agent or a mixture of agents comprising, e.g., two, three or four of the mentioned hypertrophic agents.

Chondrogenic factor - as used herein, refers to any peptide, polypeptide, protein, or any other compound or composition which induces differentiation of mesenchymal stem cells, fibroblasts and fibroblast-like cells into chondrocytes. The ability of the compound or composition to induce or stimulate production of cartilage-characteristic proteoglycans and type II collagen by differentiated cells can be determined by an *in vitro* assay (e.g. RT-PCR). Examples of chondrogenic factors are FGF (acid or basic), bone morphogenic protein (BMP), including BMP-2 and BMP-7, and GDFs. Compositions of the invention contain one or more of these chondrogenic factors, i.e. one factor or a mixture of factors comprising, e.g., two, three or four of the mentioned chondrogenic factors.

Osteogenic factor - as used herein, refers to any peptide, polypeptide, protein or any other compound or composition which induces or stimulates the formation of bone. The osteogenic factor induces differentiation of mesenchymal stem cells, fibroblasts and fibroblast-like cells into bone cells, such as osteoblasts or osteocytes. This process may be reached via an intermediary state of cartilage tissue. The bone tissue formed from bone cells will contain bone specific substances such as type I collagen fibrils, hydroxyapatite mineral and various glycoproteins and small amounts of bone proteoglycans. Examples of osteogenic factors are TGF-ß, osteogenin, bone morphogenic protein (BMP), FGF, and TGF-ß combined with epidermal growth factor (EGF). Some of these compounds are both chondrogenic and osteogenic factors. Compositions of the invention may contain one or more of these osteogenic factors, i.e. one factor or a mixture of factors comprising, e.g., two, three or four of the mentioned osteogenic factors.

Matrix - as used herein, refers to a porous composite, solid or semi-solid substance having pores or spaces sufficiently large to allow cells to populate the matrix. The term matrix includes matrix-forming materials, i.e., materials which can form matrices within a defect site in cartilage or bone. Matrix-forming materials may require addition of a polymerizing agent to form a matrix, such as adding thrombin to a solution containing fibrinogen to form a fibrin matrix. Other matrix materials include collagen, combinations of collagen and fibrin, agarose (e.g., Sepharose), and gelatin. Calcium phosphates, such as tricalcium phosphate, hydroxyapatite or other calcium salts that from solid matrices may be used alone or in combination with other matrix materials in treating defects in bones.

This invention relates to compositions for treading defects or lesions in cartilage and bone. The compositions of this invention comprise layers of flaps of synovium or peritendineum and interposed matrices containing one or more chondrogenic factors and one or more anti-hypertrophic agents.

The flaps of synovium or peritendineum are isolated from the same joint which has a defect. For inducing chondrogenesis effectively, the flaps may be treated with enzymes or punched by a needle before transplantation.

Enzymes useful to enhance chondrogenesis of the flaps of synovium or peritendineum in the methods of this invention include matrix metalloproteinases (MMPs), for example collagenase, stromelysin, matrilysin, or gelatinase, or mediators for MMP expression, such as IL-1, IL-6, TNF-α, IL-17, IL-18, or transcription factors, such as ESE-1, GADD45, DDR2, chondroitinase ABC, chondroitinase AC, or hyaluronidase. The appropriate concentration of a particular enzyme or combination of enzymes will depend on the activity of the enzyme solution.

Preferably, the flaps of synovium or peritendineum are put into a sterile solution of collagenase at an appropriate concentration, and digestion is allowed to proceed for 5-30 min. After the enzyme has treated the flaps, the enzyme solution is washed out from the flaps.

In the center of the flaps of synovium or peritendineum, it is sometimes difficult to induce chondrogenesis because of insufficient penetration by nutrition and by chondrogenic factors. To solve this problem, the flaps may be punched by a needle before transplantation. Preferably, the flaps are punched by a 18G needle several times.

The flaps of synovium or peritendineum are layered into the defect space of cartilage and bone. The layers of flaps are interposed with a matrix containing a chondrogenic factor and an anti-hypertrophic agent or containing an osteogenic factor. The flaps containing chondro- and osteo-progenitor cells, such as mesenchymal stem cells, transform into repair cartilage or bone *in situ* through stimulation by chondrogenic or osteogenic factors, respectively. These cells may also invade the interposing matrix and then transform there. Thus matrices having pores sufficiently large to allow the cells to populate the matrices are preferred.

For use in the repair of cartilage in superficial defects or the cartilage layer in a full-thickness defect, the matrix contains a chondrogenic factor which induces differentiation of chondroprogenitor cells in synovium or peritendineum flaps into chondrocytes. The matrix also contains an anti-hypertrophic agent which has biological activity that suppresses or inhibits that chondrocytes differentiate into hypertrophic chondrocytes, thereby preventing calcified tissue formation and inadequate repair of the cartilage tissue. A chondrogenic factor and an anti-hypertrophic agent are contained within or in association with a delivery system which effects release of the agents at the appropriate time to transform the chondroprogenitor cells in flaps into chondrocytes and to inhibit hypertrophic differentiation of transformed chondrocytes.

In the case of full-thickness defects that extend significantly into the underlying bone, the bone portion of the defect is preferably filled with a bone repair matrix which contains an osteogenetic factor but no anti-hypertrophic agent. If the bone defect is large, the layers of flaps of synovium or peritendineum may also be introduced into the bone defect. The flap put between bone and cartilage portion of the defect may serve as a membranous barrier to the upgrowth of bone and blood vessels into the cartilaginous defect compartment.

Matrix materials useful in the methods and compositions for filling or otherwise dressing the cartilage or bone defects may be preformed or may be formed *in situ,* for example, by polymerizing compounds and compositions. Matrices that may be preformed include fibrinogen (activated with thrombin to form fibrin in the defect or lesion), collagen (e.g., collagen gel, collagen sponges and collagen fleece), chemically modified collagen, gelatin beads or sponges, a gel-forming substance such as agarose, gelatin and any other gel-forming biodegradable material which forms a matrix with pores sufficiently large to allow chondro- and osteoprogenitor cells to populate from the flaps of synovium or peritendineum and differentiate into chondrocytes or bone cells within the matrix and which can be degraded and replaced with cartilage or bone during the repair process. In some instances, calcium phosphate containing compounds, such as tricalcium phosphate, and hydroxyapatite, as well as other calcium salts that form solid matrices, may be used alone or in combination with other biodegradable matrix materials in treating bone defects.

In one alternative, the matrix is formed using a solution of fibrinogen, to which is added thrombin to initiate polymerization shortly before use. A fibrinogen concentration of 0.5-5 mg/mL of an aqueous buffer solution may be used. Preferably, a fibrinogen solution of 1 mg/mL of an aqueous buffer solution is used. Polymerization of this fibrinogen solution in the defect area yields a matrix with a pore size sufficiently large (e.g., approximately 50-200 µm) so that chondro- or osteoprogenitor cells are free to populate the matrix and proliferate in order to fill the volume of the defect that the matrix occupies. Preferably, a sufficient amount of thrombin is added to the fibrinogen solution shortly before application in order to allow enough time for the surgeon to deposit the material in the defect area prior to completion of polymerization. Typically, the thrombin concentration should be such that polymerization is achieved within a few to several (2-4) minutes since exposure of cartilage to air for lengthy periods of time has been shown to cause damage [Mitchell et al., J. Bone Joint Surg., 71A, 89-95 (1989)]. Excessive amounts of thrombin should not be used since thrombin has the ability to cleave growth factor molecules and inactivate them. Thrombin solutions of 10-500 units per mL, and preferably 100 units per mL, of an aqueous buffer solution may be prepared for addition to the fibrinogen solution. In a preferred embodiment of this invention, approximately 20 µL of thrombin (100 U/mL) are mixed with each mL of a fibrinogen solution (1 mg/mL) approximately 200 seconds before filling the defect. Polymerization will occur more slowly if a lower concentration of thrombin is added. It will be appreciated that the amount of thrombin solution needed to achieve fibrin polymerization within 2-4 minutes can be given only approximately, since it depends upon he environmental temperature, the temperature of the thrombin solution, the temperature of the fibrinogen solution, etc. Alternatively, where convenient, the thrombin may be added by placing it on top of the matrix solution after the solution has been placed in the defect site and allowing it to diffuse through the solution. The polymerization of the thrombin-activated matrix solution filling the defect is easily monitored by observing the thrombin-induced polymerization of an external sample of the fibrinogen solution. Preferably, fibrin matrices are formed from autologous fibrinogen molecules, i.e., fibrinogen molecules derived from the blood of the same mammalian species as the species to be treated. Non-immunogenic fibrinogen from other species may also be used.

Matrices comprising fibrin and collagen or, more preferably, fibrin and gelatin may also be used in the compositions and methods. Collagenous matrices may also be used in repairing cartilage defects, including full-thickness defects. In a preferred embodiment of this invention, more solid matrices, such as those containing hydroxyapatite or tricalcium phosphate, are used in repairing the bone portion of deep full-thickness defects.

When collagen is used as a matrix material, sufficiently viscous solutions can be made, e.g., using Collagen-Vliess® ("fleece"), Spongostan®, or gelatine-blood-mixtures, and there is no need for a polymerizing agent. Collagen matrices may also be used with a fibrinogen solution activated with a polymerizing agent so that a combined matrix results.

Polymerizing agents may also be unnecessary when other biodegradable compounds are used to form the matrix. For example, Sepharose® solutions may be chosen that will be liquid matrix solutions at 39-42°C, and become solid (i.e., gel-like) at 35-38°C. The sepharose should also be at concentrations such that the gel filling the defect has a mesh size to allow osteo- or chondroprogenitor cells to freely populate the matrix and defect area.

In the compositions of this invention used in cartilage repair, one or more anti-hypertrophic agents are added to the matrix solution in an appropriate concentration range to prevent hypertrophic differentiation of chondrocytes. Anti-hypertrophic agents that may be used include, for example, TGF-ßs, PTHrP, Wnt family proteins (proteins of Wnt5a or Wnt5b gene), Smad proteins (Smad3, Smad6 and Smad7) and MRF1 (protein of Mrf1 gene). However, these particular examples are not limiting. Any compound or composition which is capable of preventing hypertrophic differentiation of chondrocytes is useful as anti-hypertrophic agents in this invention.

One or more chondrogenic factors are also present in cartilage repair matrix. Chondrogenic factors useful in the compositions and methods to promote cartilage repair include any peptide, polypeptide, protein or any other compound or composition which induces differentiation of chondroprogenitor cells in synovium or peritendineum into chondrocytes, which produce cartilage-specific proteoglycans and type II collagen. The ability of a compound or composition to induce or stimulate production of cartilage-specific proteoglycans and type II collagen in cells can be determined using assays known in the art, e.g., as described by Seyedin SM. et al., Proc Natl Acad Sci U S A, 82, 2267-71 (1985). The chondrogenic factors useful in the compositions of this invention include, for example, FGF (acid or basic), BMPs, including BMP-2 and BMP-7, and GDFs. These chondrogenic factors may be used singly or in combination. Dimers and multimers of these factors may also be used.

Where necessary, the properly timed release of the chondrogenic factor and the anti-hypertrophic agent may be achieved by packaging them in or with an appropriate delivery system. Delivery systems useful in the compositions of this invention include liposomes, bioerodible polymers, carbohydrate-based corpuscles, water-oil emulsions, fibers such as collagen which may be chemically linked to heparin sulfate proteoglycans or other such molecules to which anti-hypertrophic agents and chondrogenic factors bind spontaneously, and osmotic pumps. Delivery systems such as liposomes, bioerodible polymers, fibers with bound anti-hypertrophic agents and chondrogenic factors, and carbohydrate-based corpuscles containing the anti-hypertrophic agents and chondrogenic factors may be mixed with the matrix solution used to fill the defect. These systems are known and available in the art [see P. Johnson and J. G. Lloyd-Jones, eds., Drug Delivery Systems (Chichester, England: Ellis Horwood Ltd., 1987)]. Liposomes may be prepared according to the procedure of Kim et al., Biochem. Biophys. Acta, 728, 339-348 (1983). Other liposome preparation procedures may also be used. The timing of anti-hypertrophic agent availability should be coordinated with the release of chondrogenic factor.

In a preferred embodiment of this invention, the matrix used in cartilage repair contains TGF-ßs as an anti-hypertrophic agent packaged in a delivery system. In particular, TGF-S1 may be used as the anti-hypertrophic agent. The preferred concentration of TGF-ß1 is about 1-100 ng/mL of matrix solution. Other TGF-ß forms or polypeptides having TGF-activity may also be useful for this purpose, as well as other growth factors.

In a preferred embodiment of this invention, the matrix used in cartilage repair also contains BMPs as a chondrogenic agent packaged in a delivery system. In particular, BMP-2 may be used as the chondrogenic factor. The preferred concentration of BMP-2 is about 50-2000 ng/mL of matrix solution.

In the compositions of this invention used for bone repair of a full-thickness defect, the matrix may contain an osteogenic factors but no anti-hypertrophic agent. In some instances, a chondrogenic factor in the matrix for cartilage repair may also work as an osteogenic factor under the condition without anti-hypertrophic agent. An osteogenic factor is sequestered or packed into an appropriate delivery system within the matrix and is released as the matrix is degraded. The delivery systems used in the cartilage repair compositions are also useful in the bone repair compositions of this invention, e.g., liposomes or carbohydrate-based corpuscles (see supra). In one embodiment of this invention, the matrix used in bone repair contains BMP-2 packed in a delivery system as the osteogenic factor, at a preferable concentration of 100-2000 ng/mL of matrix solution.

Osteogenic factors useful in the bone repair compositions of this invention include any peptide, polypeptide, protein or any other compound or composition which induces differentiation of osteoprogenitor cells in flaps into bone cells, such as osteoblasts and osteocytes, which produce bone tissue. The osteogenic factors useful in this invention include proteins such as TGF-ß, osteogenin, bone morphogenic protein (BMP), FGF, and TGF-ß combined with epidermal growth factor (EGF).

The compositions hereinbefore described are useful in methods to induce cartilage or bone tissue formation at a selected site of defect in cartilage or bone tissue of an animal, in particular of a human.

The compositions of this invention allow for a method of treatment of cartilage and bone defects in animals, including humans, that is simple and is restricted in location to an affected joint area. The entire treatment may be carried out by arthroscopic, open surgical or percutaneous procedures. To carry out the methods of treating defects or lesions in cartilage and bone, a defect or lesion is identified, prepared, and filled with the flaps of synovium or peritendineum and the matrix compositions according to this invention. After the defect site is dressed with the flaps and the matrix, the joint capsule and skin incisions may be closed and the arthroscopy or open surgery terminated.

For cartilage repair, the matrix, which interposes the flaps of synovium or peritendineum, contains a chondrogenic factor at an appropriate concentration to transform chondroprogenitor cells in the flaps into chondrocytes. The matrix composition also contains an anti-hypertrophic agent to prevent hypertrophic differentiation of transformed chondrocytes. The chondroprogenitor cells are exposed to them at the appropriate time at a concentration sufficient to transform the chondroprogenitor cells into chondrocytes and to prevent hypertrophic differentiation of transformed chondrocytes. The transformed chondrocytes produce stable cartilage tissue. This step may be accomplished by including an appropriate delivery system containing the chondrogenic factor and anti-hypertrophic agent within the matrix composition as described above.

Cartilage or bone defects in animals including man are readily identifiable visually during arthroscopic examination of the joint or during simple examination of the lesion or defect during open surgery. Cartilage or bone defects may also be identified inferentially by using computer aided tomography (CAT scanning), X-ray examination, magnetic resonance imaging (MRI), analysis of synovial fluid or serum markers, or by any other procedure known in the art. Once a defect has been identified, the surgeon may select to leave the defect as is to enhance the ability of the defect site to physically retain the solutions and matrix material that are added in the treatment methods described herein. Preferably, instead of having a flat or shallow concave geometry, the defect has or is shaped to have vertical edges or is undercut in order to better retain the solutions and matrix materials added in the treatment methods described herein. The bone defect site of a full-thickness defect may be filled up to the calcified layer at the bone-cartilage interface with a bone repair matrix composition such that a flat plane is formed. The bone repair matrix composition may contain an osteogenic factor in an appropriate delivery system but no anti-hypertrophic agent. If the defect site is large, layers of flaps of synovium or peritendineum may also be introduced into the defect. The remaining cartilage portion of the defect is completely filled with layers of flaps of synovium or peritendineum and a matrix composition used to stimulate cartilage repair. The composition for cartilage repair comprises a matrix material containing a chondrogenic factor and an anti-hypertrophic agent. Anti-hypertrophic agents useful in the compositions of this invention include any agents with biological activity capable of inhibiting hypertrophic differentiation of chondrocytes. This invention contemplates that the anti-hypertrophic agent may comprise one or more molecules capable of inhibiting hypertrophic differentiation of chondrocytes.

As described in U.S. Pat. No. 5,270,300, the bone-cartilage interface of a full-thickness defect may be separated with a physical membrane, preferably a biodegradable membrane which is impermeable to cells (e.g., with pore size less than 5 µm), prior to filling the cartilage portion of a full-thickness defect. The membrane is placed over the matrix-filled bone defect, and the edges of the membrane must be sealed to the perimeter of the defect site in the region of the cartilage-bone junction to prevent vascular ingrowth into the cartilage defect area. In this method, the cells from the bone area are not readily available to populate the cartilage defect.

In a preferred method for treating a full-thickness defect, a flap of synovium or peritendineum between bone and cartilage may serve as a membranous barrier to prevent the upgrowth of bone and blood vessels into the cartilaginous defect compartment.

In another method for treating full-thickness defects, the bone-cartilage interface is separated by a transient biological membrane, created at the bone-cartilage interface by a heated instrument. A heated instrument may be applied to locations of bleeding to coagulate the blood and form a layer of precipitated protein, thus providing a biological physical barrier that prevents ingrowth of blood vessels and bone tissue formation in the defect site. Examples of heated instruments include, but are not limited to, heated scalpel blade, heated scissors or heated forceps. The instrument should be heated to a temperature of about 200°C. The heated instrument should be applied to the base of the full-thickness defect. In another embodiment, heat may be applied by a CO₂, N₂ or Neodynium-YAG laser. This heat-created transient biological membrane at the bone-cartilage interface may be employed in addition to a further biodegradable membrane and/or flaps of synovium or peritendineum serving as a membranous barrier.

Chemical measures may enhance matrix adhesion. Such measures include degrading the superficial layers of cartilage proteoglycans on the defect surface to expose the collagen fibrils of the cartilage so that they interact with the collagen fibrils of the matrix (when a collagenous matrix is used) or with the fibrin fibrils of the matrix (when a fibrin matrix is used). The proteoglycans on the surface of the cartilage not only tend to interfere with adherence of a fibrin or other biodegradable matrix to the cartilage, but also inhibit thrombin activity locally.

The surface of the defect may be dried by blotting the area using sterile absorbent tissue, and the defect volume is filled with a sterile enzyme solution for a period of 2-10 minutes to degrade the proteoglycans present on the surface of the cartilage and locally within approximately 1 to 2 µm depth measured from the surface of the defect. Various enzymes may be used, singly or in combination, in sterile buffered aqueous solutions to degrade the proteoglycans. The pH of the solution should be adjusted to optimize enzyme activity. Enzymes useful to degrade the proteoglycans in the method of this invention include chondroitinase ABC, chondroitinase AC, hyaluronidase, pepsin, trypsin, chymotrypsin, papain, pronase, stromelysin and Staph V8 protease. The appropriate concentration of a particular enzyme or combination of enzymes will depend on the activity of the enzyme solution.

Preferably, the defect is filled with a sterile solution of chondroitinase AC at a concentration of 1 U/mL, and digestion is allowed to proceed for 4 minutes. The preferred concentration of chondroitinase AC is determined according to the procedure described in Example 6. Any other enzyme used should be employed at a concentration and for a time period such that only superficial proteoglycans down to a depth of about 1-2 µm are degraded. The amount of time the enzyme solution is applied should be kept to a minimum to effect the degradation of the proteoglycans predominantly in the repair area. For chondroitinase ABC or AC at a concentration of 1 U/mL, a digestion period longer than 10 minutes may result in unnecessary and potentially harmful degradation of the proteoglycans outside the defect area. Furthermore, digestion times longer than 10 minutes contribute excessively to the overall time of the procedure. The overall time for the procedure should be kept to a minimum especially during open arthrotomy, because cartilage may be damaged by exposure to air. For these reasons, in the methods that include the step of degradation of proteoglycans by enzymatic digestion, digestion times of less than 10 minutes are preferred and digestion times of less than 5 minutes are most preferred. After the enzyme has degraded the proteoglycans at the surface of the defect, the enzyme solution should be removed from the defect area. Removal of the enzyme solution may be effected by using an aspirator equipped with a fine suction tip followed by sponging with a cotonoid. Alternatively, the enzyme solution may be removed by sponging up with a cotonoid alone. Following removal of the enzyme solution, the defect should be rinsed thoroughly, for example, three times, with sterile physiologic saline (e.g., 0.15 M NaCl). The rinsed defect site should then be dried. Sterile gauze or cotonoid may be used to dry the defect site.

The adhesion of the matrix to the cartilage at the defect site can also be enhanced by using fibrin glue, i.e., blood factor XIII or fibrin stabilization factor, to promote chemical bonding (cross-linking) of the fibrils of the matrix to the cartilage collagen fibrils on the defect surface [see Gibble et al., Transfusion, 30(8), 741-47 (1990)]. The enzyme transglutaminase may be used to the same effect. Other compounds that can promote adhesion of extracellular materials may also be used.

In order that the invention described herein may be more fully understood, the following examples are set forth. These examples are for illustrative purpose and not to be construed as limiting this invention in any manner.

### Example 1: Chondrogenic transformation of flaps of synovium in vitro

Various growth factors were tested for their usefulness in inducing chondrogenesis within flaps of synovium *in vitro.* Synovium was derived from metacarpal joints from freshly slaughtered calves, obtained from a local butcher. It was cut into small pieces (approximately 5 mm in length x 1-2 mm in width) and cultured in agarose under serum-free conditions. BMP-2 (200 ng/mL), BMP-7 (200 ng/mL) or TGF-ß1 (10 ng/mL) were added into the medium every 2 days, when the medium was changed, and the effects were monitored after 6 weeks. Chondrogenic transformation was assessed histologically and biochemically. The expression of cartilage-related genes was measured by quantitative real-time PCR.

BMP-2 and BMP-7 induced the formation of cartilaginous tissue and the expression of genes, such as collagen types II, IX and XI, and aggrecan and Sox9 that are important in the formation of cartilage tissue. However, they also increased the expression level of collagen type X gene, which is a marker of terminal (hypertrophic) differentiation of chondrocytes. TGF-ß1 induced the formation of a metachromatically stained extracellular matrix, but the gene-expression levels of collagen types II and X collagen were barely raised above the control (without growth factors) levels.

### Example 2: Chondrogenic transformation of flaps of peritendineum in vitro

The chondrogenic potential of peritendineum was tested. Peritendineum was derived from a tendon along a metacarpal joint from freshly slaughtered calves, obtained from a local butcher. It was cut into small pieces (approximately 5 mm in length x 5 mm in width) and cultured in agarose under serum-free conditions. BMP-2 (2000 ng/mL) was added into the medium every 2 days, when the medium was changed, and the effects were monitored after 6 weeks. Chondrogenic transformation was assessed histologically.

BMP-2 induced the formation of cartilaginous tissue within flaps of peritendineum.

### Example 3: Anti-hypertrophic effect of TGF-ß1 on chondrogenesis of synovial flaps in vitro

The anti-hypertrophic effect of TGF-ß1 on chondrogenesis of synovial flaps was tested. Synovium was derived from metacarpal joints from freshly slaughtered calves, obtained from a local butcher. It was cut into small pieces (approximately 5 mm in length x 1-2 mm in width) and cultured in agarose under serum-free conditions. BMP-2 (200 ng/mL) was added to induce chondrogenesis. In addition to BMP-2, TGF-ß1 (10 ng/mL) was added into the medium. These growth factors were introduced again every 2 days, when the medium was changed, and the effects were monitored after 4 weeks. Chondrogenic transformation was assessed histologically and biochemically. The expression of cartilage-related genes was measured by quantitative real-time PCR.

BMP-2 induced the formation of cartilaginous tissue in synovial flaps but also increased the expression level of collagen type X gene, which is a marker of terminal (hypertrophic) differentiation of chondrocytes. The combination of BMP-2 and TGF-ß1 enhanced the formation of cartilaginous tissue as compared to the single use of BMP-2. Furthermore, the combination inhibited the increase of the expression of collagen type X gene by BMP-2.

### Example 4: Enhancement of chondrogenesis in synovial flaps with collagenase

To enhance the chondrogenesis in synovial flaps, the effect of collagenase was tested. Synovium was derived from metacarpal joints from freshly slaughtered calves, obtained from a local butcher. It was cut into small pieces (approximately 5 mm in length x 1-2 mm in width) and cultured in agarose under serum-free conditions. Collagenase was added at a concentration of 0.3, 3 or 30 U/mL for the first 2 days of culturing. To induce chondrogenesis, BMP-2 (200 ng/mL) was added into the medium and introduced again every 2 days, when the medium was changed. The effects were monitored after 4 weeks. Chondrogenic transformation was assessed histologically.

Collagenase enhanced the volume fraction of metachomasia in synovial flaps significantly at a concentration of 30 U/mL.

### Example 5: Enhancement of chondrogenesis in synovial flaps by needle-punching

To enhance the chondrogenesis in synovial flaps, synovial flaps were punched by a needle before culturing. Synovium was derived from metacarpal joints from freshly slaughtered calves, obtained from a local butcher. It was cut into small pieces (approximately 5 mm in length x 1-2 mm in width). The flaps of synovium were punched by a 18G needle evenly across them. The punched-flaps were cultured in agarose under serum-free conditions. To induce chondrogenesis, BMP-2 (200 ng/mL) was added into the medium and introduced again every 2 days, when the medium was changed. The effects were monitored after 4 weeks. Chondrogenic transformation was assessed histologically.

The volume fraction of metachromasia was significantly higher in the punched-flaps than it in non-treated flaps.

### Example 6: Enzyme testing for proteoglycan removal

In order to promote and improve matrix adherence along superficial defect surfaces of articular cartilage tissue, proteoglycan molecules within the superficial cartilage matrix may be removed enzymatically, in order to expose the collagen fibrillar network to externally applied matrices and to migrating repair cells. Various proteases and glycosaminoglycan-degrading enzymes are suitable to be used for this purpose, but pH conditions should be controlled to provide maximal activity for each enzyme.

In this example, chondroitinase ABC (0.5-5 U/mL) and trypsin (0.5-4%) were tested for their ability to effect proteoglycan removal. Knee joints from freshly slaughtered rabbits, obtained from a local butcher, were employed. Mechanically-created superficial cartilage defects were exposed to the enzyme solutions for a period of 4 minutes. Solutions were then removed with absorbent tissue and the defect sites rinsed thoroughly with physiologic saline. Following this procedure, cartilage tissue was fixed immediately in 2% (v/v) glutaraldehyde solution (buffered with 0.05 M sodium cacodylate, pH 7.4) containing 0.7% (w/v) ruthenium hexamine trichloride (RHT) for histological examination. The post-fixation medium consisted of a 1% RHT-osmium tetroxide solution (buffered with 0.1 M sodium cacodylate). Tissue was dehydrated in a graded series of ethanol and embedded in Epon 812. Thin sections were cut, stained with uranyl acetate and lead citrate, and examined in an electron microscope. In these sections, RHT-fixed (i.e., precipitated) proteoglycans appeared as darkly-staining granules. Enzyme concentrations removing a superficial layer of proteoglycans no more than 1-2 µm in thickness were defined as optimal (deeper penetration of enzymes could affect the underlying chondrocytes). Chondroitinase ABC was found to be optimally active at a concentration of approximately 1 U/mL. Trypsin was found to be optimally active at a concentration of approximately 2.5%.

The optimal activity range for other glycosaminoglycanases or proteases may be determined in a similar manner. Any buffer may be used in conjunction with the enzyme provided that it is nontoxic and that its maximal buffering capacity occurs at a pH value close to that required for maximal enzyme activity.

### Example 7: Matrix adherence to superficial defects

The possibility of promoting matrix adhesion along defect surfaces by controlled enzyme digestion of superficial cartilage proteoglycans was investigated. Defects were created in the knee joints of three mature rabbits by cutting with a planning knife. These defects were not enzyme treated. The defects were filled with a fibrin matrix, formed by mixing 20 µL of a thrombin solution (100 U/mL aqueous buffer) approximately 200 seconds before filling the defect. The rabbits were sacrificed after 1 month and the knee joints examined to determine the extent to which the fibrin matrix had adhered to the defect site. The results were compared to those achieved in rabbits whose defects had been treated with chondroitinase ABC (1 U/mL for 4 minutes) before the defect was filled with fibrin matrix (see Examples 3, 4 and 5).

The fibrin matrices deposited in defect areas left untreated with an enzyme exhibited low affinity to adhere to the defect surface. Following enzyme treatment, the sticking capacity of the fibrin matrix (determined indirectly by measuring mechanical strength to adhere, i.e., by testing the easiness with which the matrix could be pushed away manually with the tip of a forceps and indirectly by noting the number of defects in which the matrix successfully remained sticking throughout the experiment) was significantly increased. The low affinity of matrices for the defect surfaces in the absence of enzyme treatment probably is due to a local inhibition of matrix adhesion by proteoglycan molecules and an inhibition of fibrin polymerization. Both of these effects are prevented by enzymatic removal of superficial proteoglycans along the defect surface area.

### Example 8: Application of heating procedure in full-thickness defects in articular cartilage

Full-thickness articular cartilage defects, 1 mm deep and 10 mm wide, were created in the medial condyles and patellar grooves of adult mini-pigs. Five lesions were effected in each knee joint of two mini-pigs. At each location where bleeding occurred, coagulation was induced by applying a scalpel blade heated to 220°C to the floor of defects to create a transient tissue barrier.

In one mini-pig, articular cartilage defects in one joint were filled with a cartilage repair matrix containing IGF-1 at a concentration of about 40 ng/mL of matrix volume and liposome-encapsulated TGF-ß3 at a concentration of 500 ng/mL of matrix volume. In the defect in the other joint, free and liposome-encapsulated Suramin (anti-angiogenic agent) was included in the matrix at a concentration of 10 millimolar of matrix volume. In the second mini-pig, the defects of one joint were filled with a cartilage repair matrix containing IGF-1 at a concentration of about 40 ng/mL of matrix volume and liposome-encapsulated BMP-2 at a concentration of 1000 ng/mL of matrix volume. In the defects of the other joint of the second mini-pig, Suramin was included in the matrix as described above.

The animals were sacrificed and examined eight weeks after operation and treatment. No bone tissue formed in either animal. Rather, the defect spaces were filled with articular cartilage tissue.

### Example 9: Repair of partial-thickness defect in articular cartilage using liposome-encapsulated BMP-2 and synovial flaps

BMP-2 was encapsulated in liposomes according to the method of Kim et al., Biochem. Biophys. Acta, 728, 339-348 (1983) and mixed into the fibrinogen solution (1 mg/mL). Large partial-thickness articular cartilage defects, 10 mm in length x 5 mm in wide, were created in adult goat. The thin strips (flaps) of synovial membrane were layered horizontally to defects produced in the knee following chondroitinase AC treatment and rinsing. Between these strips, a fibrinogen solution containing liposome-encapsulated BMP-2 was sandwiched. After 4-5 weeks, the strips of synovial membrane had undergone transformation into cartilage-like tissue.

## Claims

1. A composition for the treatment and repair of defects or lesions in cartilage comprising peritendineal tissue flaps or synovial tissue flaps, one or more chondrogenic factors, and one or more anti-hypertrophic agents.

2. The composition according to claim 1, wherein the chondrogenic factors are associated with a delivery system, the delivery system being contained within a matrix or matrix-forming material, and the chondrogenic factors being present at an appropriate concentration to induce chondrogenesis of peritendineal tissue or synovial tissue.

3. The composition according to claim 1, wherein the anti-hypertrophic agents are associated with a delivery system, the delivery system being contained within a matrix or matrix-forming material, and the anti-hypertrophic agent being present at an appropriate concentration to inhibit hypertrophic differentiation of the chondrocytes transformed with a chondrogenic factor.

4. The composition according to claim 1, wherein the peritendineal tissue is pretreated with enzymes or by punching with a needle.

5. The composition according to claim 1, wherein the synovial tissue is pretreated with enzymes or by punching with a needle.

6. The composition according to claim 1, wherein said chondrogenic factors are selected from BMP-2, BMP-7, and GDF-5.

7. The composition according to claim 1, wherein said anti-hypertrophic agents are selected from TGF-ßs, parathyroid hormone-related peptide (PTHrP), Wnt family proteins, Smad proteins, MRF1, prostaglandin E-2 (PGE-2), and transcription factors AP-2, delta EF-1, P300, and E2F1.

8. The composition according to claim 1 comprising layers of flaps of synovium or peritendineum with interposed layers of a cartilage repair matrix containing a chondrogenic factor and an anti-hypertrophic agent.

9. The composition according to claim 2, 3 or 8, wherein said matrix is fibrin.

10. The composition according to claim 2 or 3, wherein said delivery system is selected from the group consisting of liposomes, bioerodible polymers, collagen fibers chemically linked to heparin sulfate proteoglycans, and carbohydrate-based corpuscles.

11. The composition according to claim 4 or 5, wherein said enzymes are selected from collagenase, stromelysin, matrilysin, gelatinase, IL-1, IL-6, TNF-α, IL-17, IL-18, ESE-1, GADD45, DDR2, chondroitinase ABC, chondroitinase AC, and hyaluronidase.

12. The composition according to claim 1 comprising synovial tissue flaps, BMP-2 or BMP-7, and TGF-ß.

13. A kit of parts comprising a first composition for the treatment and repair of defects or lesions in cartilage according to anyone of claims 1 to 12 and a second composition for bone repair comprising peritendineal tissue or synovial tissue, one or more chondrogenic factors and/or one or more osteogenic factors, said second composition containing no anti-hypertrophic agents.

## Patentansprüche

1. Zusammensetzung zur Behandlung und Reparatur von Defekten oder Läsionen des Knorpelgewebes, umfassend Peritendineum-Gewebestücke oder synoviale Gewebestücke, einen oder mehrere chondrogene Faktoren sowie eine oder mehrere anti-hypertroph wirksamen Substanzen.

2. Zusammensetzung gemäss Anspruch 1, wobei die chondrogenen Faktoren mit einem Freisetzungssystem assoziiert sind, wobei das Freisetzungssystem in einer Matrix oder einem Matrix-bildenden Material enthalten ist, und wobei die chondrogenen Faktoren in einer zur Induktion einer chondrogenen Gewebedifferenzierung aus Peritendinealgewebe oder Synovialgewebe geeigneten Konzentration vorliegen.

3. Zusammensetzung gemäss Anspruch 1, wobei die anti-hypertrophen Substanzen mit einem Freisetzungssystem assoziiert sind, wobei das Freisetzungssystem in einer Matrix oder einem Matrix-bildenden Material enthalten ist, und wobei die anti-hypertrophen Substanzen in einer zur Inhibition der hypertrophen Differenzierung der mit einem chondrogenen Faktor transformierten Chondrozyten geeigneten Konzentration vorliegen.

4. Zusammensetzung gemäss Anspruch 1, wobei das Peritendineum-Gewebe mit Enzymen oder durch Einstechen mit einer Nadel vorbehandelt wurde.

5. Zusammensetzung gemäss Anspruch 1, wobei das Synovialgewebe mit Enzymen oder durch Einstechen mit einer Nadel vorbehandelt wurde.

6. Zusammensetzung gemäss Anspruch 1, wobei die besagten chondrogenen Substanzen ausgewählt sind aus BMP-2, BMP-7, und/oder GDF-5.

7. Zusammensetzung gemäss Anspruch 1, wobei die besagten anti-hypertrophen Substanzen ausgewählt sind aus den TGF-βs, parathyroid-hormonverwandten Peptiden (PTHrP), Proteinen aus der Familie Wnt, Smad-Proteinen, MRF1, Prostaglandinen (PEG-2), und den Transkriptionsfaktoren AP-2, delta EF-1, P300, und E2F1.

8. Zusammensetzung gemäss Anspruch 1, umfassend Schichten oder Stücke aus Peritendineumgewebe oder Synovialgewebe mit dazwischen angeordneten Schichten eine knorpelregenerative Matrix, welche einen chondrogenen Faktor und einen anti-hypertrophen Faktor enthält.

9. Zusammensetzung gemäss den Ansprüchen 2, 3 oder 8, wobei die besagte Matrix Fibrin ist.

10. Zusammensetzung gemäss den Ansprüchen 2 oder 3, wobei das genannte Freisetzungssystem ausgewählt ist aus der aus Liposomen, biodegradierbaren Polymeren, mit Heparinsulfatproteoglykanen vernetzten Kollagenfibrillen, und Kohlehydratpolymer- basierten Korpuskeln bestehenden Gruppe.

11. Zusammensetzung gemäss den Ansprüchen 4 oder 5, wobei die besagten Enzyme ausgewählt sind aus Kollagenase, Stromelysin, Matrilysin, Gelatinase, IL-1, IL-6, TNF-α, IL-17, IL-18, ESE-1, GADD-45, DDR-2, Chondroitinase ABC, Chondroitinase AC, und Hyaluronidase.

12. Zusammensetzung gemäss Anspruch 1, umfassend synoviale Gewebestücke, BMP-2 oder BMP-7, und TGF-β.

13. Mehrteiliges Kit, umfassend eine erste Zusammensetzung zur Behandlung und Reparatur von Defekten oder Läsionen des Knorpelgewebes nach irgendeinem der Ansprüche 1 bis 12 sowie eine zweite Zusammensetzung zur Knochengeweberegeneration umfassend Peritendinealgewebe oder Synovialgewebe, einen oder mehrere chondrogene Faktoren und/oder einen oder mehrere osteogene Faktoren besteht, wobei die besagte zweite Zusammensetzung keine anti-hypertrophen Substanzen enthält.

## Revendications

1. Une composition pour le traitement et la réparation de défauts ou lésions du cartilage comprenant des lambeaux de tissu péritendineux ou des lambeaux de tissu synovial, un ou plusieurs facteurs chondrogènes, et un ou plusieurs agents anti-hypertrophiques.

2. La composition selon la revendication 1, dans laquelle les facteurs chondrogènes sont associés à un système de délivrance, le système de délivrance étant contenu dans une matrice ou un matériau formant une matrice, et les facteurs chondrogènes étant présents à une concentration appropriée pour induire la chondrogenèse de tissu péritendineux ou de tissu synovial.

3. La composition selon la revendication 1, dans laquelle les agents anti-hypertrophiques sont associés à un système de délivrance, le système de délivrance étant contenu dans une matrice ou un matériau formant une matrice, et l'agent anti-hypertrophique étant présent à une concentration appropriée pour inhiber la différenciation hypertrophique des chondrocytes transformés avec un facteur chondrogène.

4. La composition selon la revendication 1, dans laquelle le tissu péritendineux est prétraité avec des enzymes ou perforé avec une aiguille.

5. La composition selon la revendication 1, dans laquelle le tissu synovial est prétraité avec des enzymes ou perforé avec une aiguille.

6. La composition selon la revendication 1, dans laquelle lesdits facteurs chondrogènes sont sélectionnés à partir de BMP-2, BMP-7 et GDF-5.

7. La composition selon la revendication 1, dans laquelle lesdits agents anti-hypertrophiques sont sélectionnés à partir de TGFβs, de protéine apparentée à la parathormone (PTHrP), de protéines de la famille Wnt, de protéines Smad, de MRF1, de prostaglandine E-2 (PGE-2), et de facteurs de transcription AP-2, delta EF-1, P300, et E2F1.

8. La composition selon la revendication 1 comprenant des couches de lambeaux de synoviale ou de tissu péritendineux avec des couches interposées de matrice de réparation du cartilage contenant un facteur chondrogène et un agent anti-hypertrophique.

9. La composition selon les revendications 2, 3 ou 8, dans lesquelles ladite matrice est de la fibrine.

10. La composition selon la revendication 2 ou la revendication 3, dans lesquelles ledit système de délivrance est sélectionné à partir du groupe constitué de liposomes, de polymères bioérodables, de fibres de collagène chimiquement liées à des protéoglycanes à héparane sulfate, et de corpuscules à base d'hydrates de carbone.

11. La composition selon la revendication 4 ou la revendication 5, dans lesquelles lesdites enzymes sont sélectionnées à partir de collagénase, de stromélysine, de matrilysine, de gélatinase, d'IL-1, d'IL-6, de TNF-α, d'IL-17, d'IL-18, d'ESE-1, de GADD45, de DDR2, de chondroitinase ABC, de chondroitinase AC, et de hyaluronidase.

12. La composition selon la revendication 1 comprenant des lambeaux de tissu synovial, du BMP-2 ou du BMP-7, et du TGF-β.

13. Un kit d'éléments comprenant une première composition pour le traitement et la réparation de défauts ou lésions du cartilage selon l'une quelconque des revendications 1 à 12 et une seconde composition pour la réparation de l'os comprenant le tissu péritendineux ou le tissu synovial, un ou plusieurs facteurs chondrogènes et/ou un ou plusieurs facteurs ostéogènes, ladite seconde composition ne contenant pas d'agents anti-hypertrophiques.
